# EUROPÄISCHE PATENTSCHRIFT

(11) **EP 1 000 587 B1**
(45) Veröffentlichungstag und Bekanntmachung des Hinweises auf die Patenterteilung: **18.10.2006**
(21) Anmeldenummer: 99122713.3
(22) Anmeldetag: 16.11.1999
(51) Int. Cl.: A61B 18/00

(54) **Absaugvorrichtung**
Suction device
Dispositif d'aspiration

(30) Priorität: 16.11.1998 DE 19852705
(43) Veröffentlichungstag der Anmeldung: 17.05.2000
(73) Patentinhaber: ATMOS MEDIZINTECHNIK GmbH & Co., 79853 Lenzkirch (DE)
(72) Erfinder: Möller, Mario, 79843 Löffingen (DE); Rheiner, Norbert, 79848 Bonndorf (DE)
(74) Vertreter: Patentanwälte Westphal, Mussgnug & Partner

(56) Entgegenhaltungen:
- DE-A- 4 206 241
- GB-A- 2 010 794
- GB-A- 2 300 368
- US-A- 3 862 420
- US-A- 5 423 779

## Beschreibung

Die Erfindung betrifft eine Absaugvorrichtung gemäß dem Oberbegriff des Anspruchs 1.

Bei einer Behandlung mit thermischen Chirurgiegeräten wie Elektro-Chirurgiegeräten oder dem medizinischen Laser (z.B. Erbium: YAG, CO₂) entstehen flüchtige Pyrolyseprodukte (Gase und Aerosole), welche im Falle der Laserchirurgie als "Laser Smoke" bezeichnet werden. Störende Gase können auch bei der Verwendung von zweikomponentigen Zementen oder Klebstoffen z.B. in der Implantat-Chirurgie entstehen.

Die bekannten Vorrichtungen zum Absaugen dieser Pyrolyseprodukte bzw. des "Laser Smokes" sind aus dem Stand der Technik in einer Vielzahl von Abwandlungen bekannt.

So ist beispielsweise aus der US 4,963,134 eine Chirurgievorrichtung mit der Absaugvorrichtung bekannt. Die Absaugvorrichtung weist eine Filtervorrichtung auf, die einer Absaugdüse und einem daran angeschlossenen Schlauch nachgeschaltet ist. Eine erste Filterstufe (Vorfilter) weist einen Feuchtigkeitsfilter und einen Aktivkohlefilter auf. In einer zweiten Filterstufe wird eine Chemikalie in eine Kammer zu den abgesaugten Dämpfen eingebracht, um diese zu desinfizieren, bevor die so behandelten Dämpfe über einen Partikelfilter, einen sogenannten High-Efficiency-Particle-Absorbing (HEPA)-Filter nach außen abgegeben werden.

In der US 5,709,675 ist eine Absaugvorrichtung mit einer Filtervorrichtung beschrieben, welche aus zwei Filterstufen besteht. Der erste Filter ist direkt an der Lufteinlaßöffnung angeordnet und besteht vorzugsweise aus einem Material mit besonderen Adsorptionseigenschaften. Als Filtermaterial eignet sich insbesondere Aktivkohle oder auch Polyester. Nach dieser Druckschrift kann der Filter aber auch ein Material mit außergewöhnlichen Absorptionseigenschaften (HEPA-Filter) beinhalten oder ein Fasermaterial (Fiber-Typ-Filter). Ein zweiter Filter ist vorzugsweise dazu vorgesehen, Stäube oder Partikel aufzufangen, die den ersten Filter passieren. Dieser Filter basiert bevorzugt auf den guten Absorptionseigenschaften des Filtermaterials.

Aus den oben angegebenen Druckschriften US 4,963,134 und US 5,709,675 sind also Absaugvorrichtungen mit mehrstufigen Filtersystemen bekannt, wobei die Einzelfilter nach den Prinzipien der Adsorption und der Absorption arbeiten. Absorptionsfilter oder Partikelfilter sind weitgehend optimiert. Adsorptionsfilter, wozu unter anderem die oben angegebenen Aktivkohle-Filter gehören, wirken vor allem durch die sog. Van-der-Waalschen-Kräfte. Gasmoleküle lagern sich an der sehr großen inneren Oberfläche der aktivierten Kohle an. Diese Filter, die mit Standard-Aktivkohle gefüllt sind, sind jedoch nicht in der Lage andere Gase als die sog. schnellflüchtigen organischen Gase (Volatile organic compounds = VOC's) wie Butanale, Pyrazine etc. wirkungsvoll zu filtern. Diese Fraktion der VOC's macht aber nur ca. 3% der Gesamt-Gaskonzentration im Pyrolyseprodukt von chirurgischem Laser bzw. Hochfrequenz-Chirurgiegerät aus.

Neben CO₂ welches 91% der Gesamtgaskonzentration ausmacht, entstehen aber auch toxische Gase welche in größeren Konzentrationsanteilen vorliegen. Diese werden aber von den bisherigen Filtersystemen nicht erfaßt.

Nachteilig bei diesen Filtersystemen ist auch, dass ein Wechsel der Filter nach einer oft willkürlichen Zeitvorgabe des Herstellers erfolgt, wobei für die unterschiedlichen Filter in der Regel unterschiedliche Haltbarkeitszeiträume gelten. In der Praxis erfolgt dadurch ein Filterwechsel im Anschluss an eine subjektive Effizienzbewertung der Filterwirkung, d.h. nach einer geruchssensorischen Wahrnehmung von einzelnen VOC's. Dies ist möglich, da in diesen VOC's die sehr geruchintensiven Pyrazine, Butanale, und andere Verbindungen enthalten sind.

Bei vielen bekannten Gasen ist die geruchssensorische Wahrnehmung, bspw. durch das OP-Personal, nicht gegeben. Solche Gase werden wegen des überlagerten starken Geruchsprofils mancher VOC's nicht eindeutig durch die Nase wahrgenommen. Um so wichtiger wird hier die Beseitigung der ins OP-Feld eingebrachten Gasfraktionen. Zu diesem Zweck sehen manche Filtersysteme eine Lebensdauerüberwachung der Filter vor.

Um den Filter in seiner Lebensdauer komfortabel überwachen zu können, speichern manche Absaugsysteme die bisherige Nutzungsdauer im Absauggerät ab. Von dieser Nutzungsdauer kann die Restkapazität des Filters abgeleitet und auch dem Anwender angezeigt werden.

Das Problem, das hier entstehen kann, liegt im fehlenden Erkennen der tatsächlichen Filterkapazität. Bei manchen Systemen führt schon der Vorgang des Filter-Entfernens und des Wiedereinsetzens desselben Filters zu einem Rücksetzen der Lebenszeit. Das Gerät zeigt dann einen neuen Filter an, obwohl dies nicht zutrifft. Manche Systeme erfordern das manuelle Rücksetzen der Standzeit durch den Anwender, was leicht zu naheliegendem Mißbrauch führen kann.

Nachteilig bei den aus dem Stand der Technik bekannten Filtersystemen von der in der US-Patentschrift US 4,963,134 beschriebenen Gattung ist weiterhin, dass zur Desinfektion der zur behandelnden Dämpfe in der Regel toxische Chemikalien eingesetzt werden. Solche Chemikalien weisen aufgrund ihrer Toxizität eine allgemein biozide Wirkung auf, so dass Filter auf der Basis solcher humantoxischer Chemikalien stets als Sondermüll zu behandeln sind.

Der Erfindung liegt somit die Aufgabe zugrunde, eine Absaugvorrichtung mit einer Filtervorrichtung zu schaffen, welche die oben angegebenen Nachteile nicht mehr aufweist. Eine solche Absaugvorrichtung soll eine oder mehrere Filter aufweisen, welche speziell auf einen Einsatz in der Laserchirurgie abgestimmt sind, mit dem Ziel der Minimierung einer gesundheitlichen Gefährdung des behandelnden Chirurgen und des Chirurgiepersonals.

Diese Aufgabe wird durch eine Absaugvorrichtung mit den Merkmalen des kennzeichnenden Teils des Anspruch 1 erfindungsgemäß gelöst.

Vorteilhafte Ausführungen und Weiterbildungen der Erfindung sind in den Unteransprüchen angegeben.

Die Erfindung liegt in der Verwendung eines Mehrstufen-Gasfiltersystem in Verbindung mit einer Einrichtung, die zur Reinigung maschinell angesaugter Luft dient. Sie bezieht sich also auf ein Filtersystem mit einem Grobfilter als Vorfilter, welcher vorzugsweise hydrophobe Oberflächen aufweist und der als Kondensationsfläche und Prallplatte wirkt, einem Partikelfilter, einem VOC-Filter und mindestens einen Zusatz-Gasfilter, der in Verbindung mit dem Standard-Aktivkohlefilter verwendet wird. Diese Zusatz-Gasfilter sind im Besonderen ein Kohlenmonoxidfilter, ein Blausäurefilter und ein Ammoniakfilter.

Durch ein Abfiltern der giftigen Gase Kohlenmonoxid (CO), Blausäure (HCN) und Ammoniak (NH₃) die etwa 6% der Gesamtgaskonzentration ausmachen und die zu annähernd gleichen Anteilen in dem "Laser-Smoke" vorliegen, wird eine unmittelbare gesundheitsschädliche Wirkung weitgehend ausgeschlossen.

Die Erfindung sieht vor, dass der Ammoniakfilter mit speziell imprägnierter aktivierter Kohle ausgeführt ist. Diese Kohle wird beispielsweise mit Phosphorsäure behandelt, um damit die Eigenschaft sehr guter Adsorptionsfähigkeiten gegenüber NH₃ zu erhalten.

Für den Blausäurefilter erweist es sich erfindungsgemäß als besonders vorteilhaft, wenn die aktivierte Kohle für diesen Blausäurefilter bspw. mit Platin, Zinkoxid, Chrom, Molybdän, anderen Schwermetallen oder aber mit basischen Imprägnierungen wie Kaliumcarbonat o.ä. behandelt wird.

Das Gas CO kann nur mit Hilfe eines speziellen Katalysators beseitigt werden, nicht jedoch mit Aktivkohle. Die Filtereffizienzen sind mit genormten Prüfverfahren, wie z.B. in EN 141 beschrieben, feststellbar.

Eine besonders effiziente Filterwirkung wird dann erzielt, wenn die zusätzlichen Filter zur Entfernung der giftigen Gase Kohlenmonoxid, Blausäure und Ammoniak den Vorfilter, den Partikelfilter und dem VOC-Filter nachfolgend angeordnet sind.

Da gerade der Partikelfilter in der Gefahr steht von Mikroorganismen wie Bakterien, Viren und Aspergillen durchwachsen zu werden, ist es wichtig, zuverlässig dem Anwender zu signalisieren welchen Nutzungsgrad die Filtereinheit erreicht hat. Eine Überwachung des Momentanzustandes ist insbesondere auch bei Beschaffungsproblemen zwingend. Es ist wichtig zu wissen, wie der Nutzungsgrad eines Filters z. Zt. aussieht, um auch in diesen Fällen eine Gesundheitsgefährdung des Personals sicher auszuschließen.

Dieses Problem wird erfindungsgemäß dadurch beseitigt, dass an oder in der Filtereinheit eine Identifikations-Kennung und/oder ein Standzeit-Speicher angebracht wird.

Die Identifikations-Kennung ermittelt den Filter als Original-Ersatzteil und verhindert gleichzeitig den Nachbau evtl. nicht gleichwertiger Plagiate. Die Kennung erhält einen speziellen Code, der mittels eines mathematischen Algorithmus' generiert wurde. Nur Filter mit einem derartig erzeugten Code werden vom Absaugsystem als originär erkannt und akzeptiert. Die Kennung kann elektronisch abgelegt werden in einem Lese- bzw. Schreib-/Lesespeicher, wie zum Beispiel einem ROM, RAM bzw. einem EEPROM. Möglich ist auch eine Kennung mittels eines Aufdrucks als Zeichen wie z.B. einem Barcode, oder eine sonstige Kennung wie bspw. eine magnetische oder farbliche Kennung.

Der Standzeit-Speicher befindet sich ebenfalls am oder im Filter und ist in jedem Fall als Schreib-/Lesespeicher ausgeführt. Der Speicher kann elektronisch, magnetisch oder auf eine andere Weise ausgeführt sein.

Um das Durchwachsen und die Besiedelung von Mikroorganismen auf der Partikelfilter-Oberfläche zu mindern, ist die Filterfläche mikrobiozid behandelt. Während manche Hersteller hier toxische Chemikalien einsetzen, liegt die Erfindung in dem Einsatz von Verbindungen, die mit dem Hausmüll entsorgt werden können und lediglich eine mikrobiozide aber keine allg. biozide Wirkung aufweisen.

Eine Gesundheitsgefährdung des Bedien- und Wartungspersonals kann damit weitgehend ausgeschlossen werden, ohne dass besondere Vorsichtmaßnahmen getroffen werden müssen. Ein positiver Nebeneffekt ergibt sich auch dadurch, dass eine umweltfreundliche Entsorgung möglich ist, die ohne eine spezielle Behandlung oder eine Lagerung auf Sondermülldeponien auskommt.

Stoffe, die einen mikrobioziden aber keinen humantoxischen Effekt besitzen, sind zum Beispiel Silberionen, Kupferionen oder allg. Mineralverbindungen wie z.B. Aluminiumverbindungen.

In einer besonders vorteilhaften Ausführung der Erfindung sind die Filterwirkungen mindestens zweier Einzelfilter im Idealfall aber aller Einzelfilter so auf die Zusammensetzung des Pyrolysegases abgestimmt, daß deren Lebensdauern zumindest annähernd identisch sind und somit gleichzeitig gewechselt werden können. Die Filterwirkungen der Einzelfilter sind also so aufeinander abgestimmt, daß bei einer Pyrolysegaszusammensetzung von 91% CO₂, 2% CO, 2% NH₃, 2% HCN und 3% VOCs die Filterwirkung des VOC-Filters, des CO-Filters, des NH₃-Filters und des HCN-Filters zur selben Zeit nachläßt. Eine modulare Bauweise wird dadurch ermöglicht, so daß das Filtersystem komplett nach Ablauf der Lebensdauer ausgewechselt werden kann.

Ein Ausführungsbeispiel der Erfindung ist in der Zeichnung dargestellt und wird im Folgenden näher beschrieben. Es zeigt:

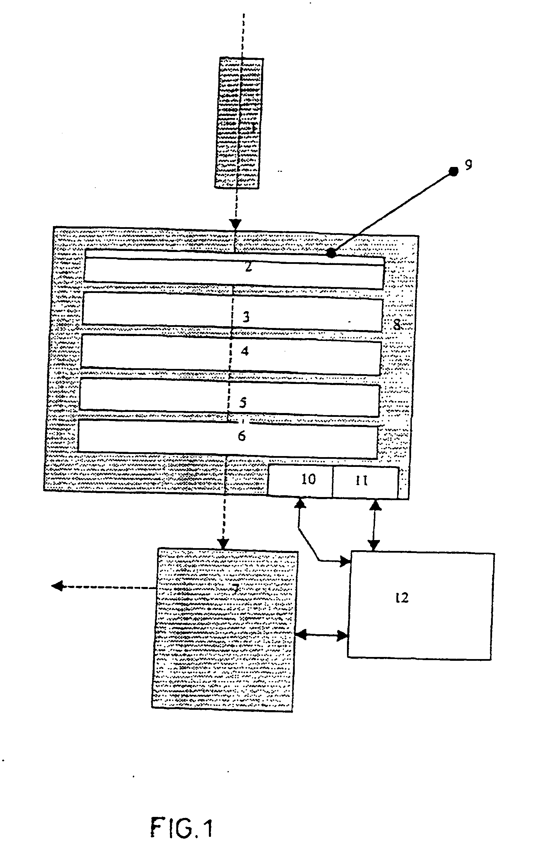

- Fig. 1: eine schematische Darstellung einer erfindungsgemäßen Absaugvorrichtung.

Figur 1 ist zu entnehmen, dass eine solche erfindungsgemäße Absaugvorrichtung beispielsweise aus einem Vorfilter 1, einem Hauptfilter in einem Hauptfiltergehäuse 8, einer Vakuumpumpe 7 und einer Steuerungs- und Überwachungseinheit 12 besteht.

Der "Laser-Smoke" wird zunächst über den Vorfilter 1 dem Hauptfilter im Hauptfiltergehäuse 8 zugeführt und dann über die Vakuumpumpe 7, an deren Austrittsöffnung die gefilterten Dämpfe/Luft austreten, abgesaugt. In der Figur 1 ist der Luftweg durch gestrichelte Pfeile angedeutet.

In dem Hauptfiltergehäuse 8 befinden sich in der Richtung des Luftweges zunächst eine mikrobiozide Behandlungsstufe 9, welche direkt dem Partikelfilter 2 zugeordnet ist. Auf den Partikelfilter 2 folgt ein VOC-Filter 3. An diesen VOC-Filter 3 schließen sich in beliebiger Reihenfolge ein CO-Filter 4, ein HCN-Filter 5 und ein NH₃-Filter 6 an, dessen Austrittsöffnung wiederum an die Vakuumpumpe 7 anschließt.

In dem Hauptfiltergehäuse 8 sind im Beispiel eine Identifikations-Kennung 10 und ein Standzeit-Speicher 11 integriert. Sowohl diese Identifikations-Kennung 10 als auch der Standzeit-Speicher 11 sind genauso wie die Vakuumpumpe 7 über Daten- und/oder (elektrische) Versorgungsleitungen mit der Steuerungs- und Überwachungseinheit 12 verbunden.

Der Standzeit-Speicher ist zur luftstromabhängigen Lebensdauerbewertung des Filters und zur Anzeige der Restkapazität vorgesehen, die Identifikations-Kennung zum Schutz des Anwenders vor dem Angebot nicht gleichwertiger Plagiate. Beide Informationen, genauso wie die Saugleistung der Vakuumpumpe, werden über die Datenleitung der Steuerungs- und Überwachungseinheit 12 zugeführt, in welcher die Daten ausgewertet werden und der Momentannutzungsgrad der Filter zur Anzeige gebracht wird.

### Bezugszeichenliste

- 1: Vorfilter
- 2: Partikelfilter
- 3: VOC-Filter
- 4: CO-Filter
- 5: HCN-Filter
- 6: NH₃-Filter
- 7: Vakuumpumpe
- 8: Hauptfiltergehäuse
- 9: Mikrobiozide Behandlungsstufe
- 10: Identifikations-Kennung
- 11: Standzeit-Speicher
- 12: Steuerungs- und Überwachungseinheit

## Patentansprüche

1. Absaugvorrichtung, welche luftgetragene oder luftgelöste Abfallprodukte einer chirurgischen Behandlung mit einem stromgetriebenen Chirurgieinstrument insbesondere mit einem Laserskalpell absaugt, mit einer Filtereinheit, die einen Vorfilter (1), einen Partikelfilter (2) und einen VOC-Filter (3) aufweist,
**dadurch gekennzeichnet, daß** die Filtereinheit einen oder mehrere zusätzliche Filter (4, 5, 6) zur Beseitigung von Kohlenmonoxid (CO), Blausäuregasen (HCN) und/oder von Ammoniakgasen (NH₃) aufweist.

2. Absaugvorrichtung nach Anspruch 1,
**dadurch gekennzeichnet, daß** diese zusätzlichen Filter dem Vorfilter (1), dem Partikelfilter (2) und dem VOC-Filter (3) nachfolgend angeordnet sind.

3. Absaugvorrichtung nach einem der vorstehenden Ansprüche,
**dadurch gekennzeichnet, daß** der zusätzliche Filter (5) zur Beseitigung von Blausäuregasen (HCN) ein Aktivkohlefilter darstellt, der mit Platin, Zinkoxid, Chrom, Molybdän, oder anderen Schwermetallen behandelt wurde.

4. Absaugvorrichtung nach einem der vorstehenden Ansprüche,
**dadurch gekennzeichnet, daß** der zusätzliche Filter (5) zur Beseitigung von Blausäuregasen (HCN) ein Aktivkohlefilter darstellt, der mit einer basischen Imprägnierung wie Kaliumcarbonat behandelt wurde.

5. Absaugvorrichtung nach einem der vorstehenden Ansprüche,
**dadurch gekennzeichnet, daß** der zusätzliche Filter (6) zur Beseitigung von Ammoniakgasen (NH₃) ein Aktivkohlefilter darstellt, der mit Phosphorsäure behandelt wurde.

6. Absaugvorrichtung nach einem der vorstehenden Ansprüche,
**dadurch gekennzeichnet, daß** vor dem Vorfilter (1), dem Partikelfilter (2), dem VOC-Filter (3) und dem oder den zusätzlichen Filtern (4, 5, 6) eine mikrobiozide Behandlungsstufe (9) vorgesehen ist.

7. Absaugvorrichtung nach einem der vorstehenden Ansprüche,
**dadurch gekennzeichnet, daß** die mikrobiozide Behandlungsstufe (9) im Oberflächenbereich Silberionen, Kupferionen, Mineralverbindungen wie Aluminiumverbindungen oder deren Ionen aufweist.

8. Absaugvorrichtung nach einem der vorstehenden Ansprüche,
**dadurch gekennzeichnet, daß** die Filter (4, 5, 6) und die mikrobiozide Behandlungstufe (9) zu einer Hauptfilterstufe (8) zusammengefaßt sind und diese mit einem Standzeit-Speicher (11) und/oder mit einer Identifikations-Kennung (10) versehen ist.

9. Absaugvorrichtung nach einem der vorangegangenen Ansprüche,
**dadurch gekennzeichnet, daß** die Filterwirkungen mindestens zweier Einzelfilter (1, 2, 3, 4, 5, 6, 9) so auf die Zusammensetzung des zu filternden Pyrolysegases abgestimmt sind, daß die Lebensdauer dieser Einzelfilter (1, 2, 3, 4, 5, 6, 9) zumindest annähernd identisch ist.

10. Absaugvorrichtung nach Anspruch 9,
**dadurch gekennzeichnet, daß** bei einer Pyrolysegaszusammensetzung von 91% CO₂, 2% CO, 2% NH₃ und 3% VOCs die Filterwirkung des VOC-Filters (3), des CO-Filters (4), des NH₃-Filters (6) und des HCN-Filters (5) zur selben Zeit nachläßt.

11. Absaugvorrichtung nach einem der vorangangenen Ansprüche,
**dadurch gekennzeichnet, daß** mindestens zwei Einzelfilter (1, 2, 3, 4, 5, 6, 9) zu einem Modul zusammengefaßt sind.

## Claims

1. Suction device which sucks up air-borne or air-dissolved waste products from a surgical treatment using a power-operated surgical instrument, in particular using a laser scalpel, said suction device having a filter unit which comprises a pre-filter (1), a particle filter (2) and a VOC filter (3), **characterised in that** the filter unit comprises one or more additional filters (4, 5, 6) for removing carbon monoxide (CO), hydrocyanic acid gases (HCN) and/or ammonia gases (NH₃).

2. Suction device according to Claim 1, **characterised in that** these additional filters are arranged downstream of the pre-filter (1), the particle filter (2) and the VOC filter (3).

3. Suction device according to one of the preceding claims, **characterised in that** the additional filter (5) for removing hydrocyanic acid gases (HCN) is an active carbon filter which has been treated with platinum, zinc oxide, chromium, molybdenum or other heavy metals.

4. Suction device according to one of the preceding claims, **characterised in that** the additional filter (5) for removing hydrocyanic acid gases (HCN) is an active carbon filter which has been treated by impregnation with a base, such as potassium carbonate.

5. Suction device according to one of the preceding claims, **characterised in that** the additional filter (6) for removing ammonia gases (NH₃) is an active carbon filter which has been treated with phosphoric acid.

6. Suction device according to one of the preceding claims, **characterised in that** a microbiocidal treatment stage (9) is provided upstream of the pre-filter (1), the particle filter (2), the VOC filter (3) and the additional filter or filters (4, 5, 6).

7. Suction device according to one of the preceding claims, **characterised in that** the microbiocidal treatment stage (9) comprises, in the surface region, silver ions, copper ions, mineral compounds such as aluminium compounds or ions thereof.

8. Suction device according to one of the preceding claims, **characterised in that** the filters (4, 5, 6) and the microbiocidal treatment stage (9) are combined to form a main filter stage (8), and the latter is provided with a service life memory (11) and/or with an identifier (10).

9. Suction device according to one of the preceding claims, **characterised in that** the filter effects of at least two individual filters. (1, 2, 3, 4, 5, 6, 9) are adapted to the composition of the pyrolysis gas to be filtered, in such a way that the service life of these individual filters (1, 2, 3, 4, 5, 6, 9) is at least approximately identical.

10. Suction device according to Claim 9, **characterised in that**, for a pyrolysis gas composition of 91% CO₂, 2% CO, 2% NH₃ and 3% VOCs, the filter effect of the VOC filter (3), of the CO filter (4), of the NH₃ filter (6) and of the HCN filter (5) ends at the same time.

11. Suction device according to one of the preceding claims, **characterised in that** at least two individual filters (1, 2, 3, 4, 5, 6, 9) are combined to form a module.

## Revendications

1. Dispositif d'aspiration des déchets, portés par l'air ou dissous dans l'air, lors d'une manipulation chirurgicale avec un instrument chirurgical à commande électrique, en particulier un scalpel à laser, ce dispositif comportant un ensemble de filtration qui présente un pré-filtre (1), un filtre à particules (2) et un filtre à VOC (3),
**caractérisé en ce que**
l'ensemble de filtration présente un ou plusieurs filtres supplémentaires (4, 5, 6) pour éliminer le monoxyde de carbone (CO), les gaz d'acide cyanhydrique (HCN) et/ou les gaz ammoniacaux (NH₃).

2. Dispositif d'aspiration selon la revendication 1,
**caractérisé en ce que**
ces filtres supplémentaires sont disposés après le pré-filtre (1), le filtre à particules (2) et le filtre à VOC (3).

3. Dispositif d'aspiration selon l'une des revendications précédentes,
**caractérisé en ce que**
le filtre supplémentaire (5) destiné à l'élimination de gaz d'acide cyanhydrique (HCN) est un filtre à charbon actif qui a été traité avec du platine, de l'oxyde de zinc, du chrome, du molybdène ou d'autres métaux lourds.

4. Dispositif d'aspiration selon l'une des revendications précédentes,
**caractérisé en ce que**
le filtre supplémentaire (5) destiné à l'élimination de gaz d'acide cyanhydrique (HCN) est un filtre à charbon actif qui a été traité avec une imprégnation basique comme le carbonate de potassium.

5. Dispositif d'aspiration selon l'une des revendications précédentes,
**caractérisé en ce que**
le filtre supplémentaire (6) destiné à l'élimination des gaz ammoniacaux (NH₃) est un filtre à charbon actif qui a été traité avec de l'acide phosphorique.

6. Dispositif d'aspiration selon l'une quelconque des revendications précédentes,
**caractérisé par**
un étage de traitement antimicrobien (9) avant le pré-filtre (1), le filtre à particules (2), le filtre à VOC (3) et le ou les filtres supplémentaires (4, 5, 6).

7. Dispositif d'aspiration selon l'une des revendications précédentes,
**caractérisé en ce que**
l'étage de traitement antimicrobien (9) présente, en surface, des ions d'argent, des ions de cuivre, des composés minéraux tels que des composés d'aluminium ou leurs ions.

8. Dispositif d'aspiration selon l'une des revendications précédentes,
**caractérisé en ce que**
les filtres (4, 5, 6) et l'étage de traitement antimicrobien (9) sont regroupés en un étage de filtration principale (8) pourvu d'une mémoire de durée de vie (11) et/ou d'un code d'identification (10).

9. Dispositif d'aspiration selon l'une des revendications précédentes,
**caractérisé en ce que**
les actions filtrantes d'au moins deux filtres individuels (1, 2, 3, 4, 5, 6, 9) sont adaptées à la composition du gaz de pyrolyse filtrant de manière à ce que la durée de vie de ces filtres individuels (1, 2, 3, 4, 5, 6, 9) soit au moins approximativement identique.

10. Dispositif d'aspiration selon la revendication 9,
**caractérisé en ce que**
l'action filtrante du filtre à VOC (3), du filtre à CO (4), du filtre à NH₃ (6) et du filtre à HCN (5) diminue en même temps pour une composition des gaz pyrolyse contenant 91 % de CO₂, 2 % de CO, 2 % de NH₃ et 3 % de VOC.

11. Dispositif d'aspiration selon l'une des revendications précédentes,
**caractérisé en ce qu'**
au moins deux filtres individuels (1, 2, 3, 4, 5, 6, 9) sont regroupés dans un module.
